# EUROPÄISCHE PATENTSCHRIFT

(11) **EP 0 530 697 B1**
(45) Veröffentlichungstag und Bekanntmachung des Hinweises auf die Patenterteilung: **08.05.1996**
(21) Anmeldenummer: 92114715.3
(22) Anmeldetag: 28.08.1992
(51) Int. Cl.: A61K 6/04

(54) **Verwendung einer Palladiumlegierung für mit Dentalkeramik verblendbaren Zahnersatz**
Use of palladium alloy for fusion to a dental ceramic
Utilisation d'un alliage de palladium pour fusion sur une céramique dentaire

(30) Priorität: 06.09.1991 DE 4129592; 11.08.1992 DE 4226484
(43) Veröffentlichungstag der Anmeldung: 10.03.1993
(73) Patentinhaber: Degussa Aktiengesellschaft, D-60311 Frankfurt (DE)
(72) Erfinder: Groll, Werner, Dr. Dipl.-Ing., W-8755 Alzenau (DE); Hathaway, Doris, W-6450 Hanau 7 (DE); Kempf, Bernd, Dr. Dipl.-Ing., W-6463 Freigericht (DE); Schöck, Gernot, Dipl.-Ing., W-6454 Bruchköbel (DE)

(56) Entgegenhaltungen:
- WO-A-90/08650
- DE-A- 3 414 575
- DE-A- 3 830 666
- US-A- 4 539 176

## Beschreibung

Die Erfindung betrifft die Verwendung von Palladiumbasis-Legierungen zur Herstellung von festsitzendem und herausnehmbaren, mit Dentalkeramik verblendbaren Zahnersatz. Festsitzender und herausnehmbarer Zahnersatz wird vorwiegend aus korrosionsbeständigen, biokompatiblen Edelmetallegierungen nach dem sogenannten Wachsausschmelzverfahren hergestellt, wobei das gegossene Objekt anschließend häufig mit Dentalkeramik verblendet wird, um ein dem natürlichen Zahn entsprechendes Aussehen zu erzielen. Hierfür müssen die Legierungen neben einer ausreichenden Festigkeit noch spezielle, auf die Dentalkeramik abgestimmte Eigenschaften besitzen, wie thermischen Ausdehnungskoeffizienten, Schmelzintervall und Haftung zwischen Keramik und Legierung.

Hochgoldhaltige Legierungen, wie sie z.B. in den DE-PSen 11 83 247 und 15 33 233 beschrieben sind, eignen sich für diesen Zweck besonders gut. Wegen des hohen Preises von Gold ist jedoch in letzter Zeit verstärkt nach preiswerten Alternativen zu den hochgoldhaltigen Legierungen gesucht worden. Im Bereich der Edelmetalle bietet sich als Ersatz das Palladium wegen seines relativ günstigen Preises, seiner gegenüber Gold deutlich verringerten Dichte und seiner dem Gold vergleichbaren Korrosions- bzw. Mundbeständigkeit an.

Die bisher entwickelten Aufbrennlegierungen, die Palladium als wesentliches Legierungselement enthalten, lassen sich grob in drei Gruppen einteilen.

Die erste Gruppe besteht aus Gold-Palladiumlegierungen, die üblicherweise silberfrei sind oder nur geringe Mengen an Silber enthalten. Typische Vertreter sind in den DE-PSen 345 06 712 und 29 44 755 beschrieben. Durch den recht hohen Goldanteil sind diese Legierungen aber immer noch relativ teuer.

Die zweite Gruppe besteht aus im allgemeinen silberfreien Palladiumbasis-Legierungen, wie sie z.B. in den DE-PSen 33 16 595, 33 04 183, 35 22 523 und 32 47 398 beschrieben sind. Ihre Hauptlegierungselemente sind Kupfer und Gallium. Weitere häufig verwendete Legierungselemente in diesen Systemen sind Zinn, Indium und Kobalt. Im Vergleich zu den hochgoldhaltigen Aufbrennlegierungen sind sie jedoch schwieriger zu verarbeiten und reagieren empfindlicher auf Verarbeitungsfehler. Die Legierungen sind schwieriger lötbar und sie nehmen im schmelzflüssigen Zustand Kohlenstoff auf, so daß sie nur in Keramiktiegeln aufgeschmolzen werden dürfen, um Blasenbildungen in den aufgebrannten keramischen Massen zu vermeiden. Alle diese Legierungen weisen darüberhinaus sehr dunkle bis schwarze Oxide auf, die sich während des Keramikbrandes bilden. Dadurch entstehen im Randbereich der Verblendungen dunkle Säume, die das ästhetische Erscheinungsbild des Zahnersatzes beeinträchtigen.

In der US-PS 4,539,176 wird eine silberfreie Palladiumlegierung beschrieben, die neben Palladium 10 bis 40 Gew. % Gold, 3 bis 8 Gew. % Gallium, 0,5 bis 10 Gew. % Indium und/oder Zinn und 0,1 bis 1,5 Gew. % Ruthenium, Iridium oder Rhenium enthält. Diese Legierung hat sich aber nicht als brauchbar erwiesen.

Die dritte Gruppe stellen die Palladium-Silber-Legierungen dar. Sie sind in ihrem Verarbeitungsverhalten zwischen den hochgoldhaltigen und den silberfreien Palladiumlegierungen anzusiedeln. Die typische Zusammensetzung solcher Legierungen ist in der "Übersicht über die Dental-Edelmetallegierungen und Dental-Nichtedelmetallegierungen in der Bundesrepublik Deutschland" herausgegeben vom Forschungsinstitut für die zahnärztliche Versorgung (FZV), Stand 1. Juli 1986, Seite 31/32, zu finden. Neben Palladium und Silber enthalten diese Legierungen vorwiegend Zinn, Indium und Zink, vereinzelt auch Kupfer oder Gallium als weitere Legierungselemente. Im allgemeinen ist die Oxidfarbe dieser Legierungen deutlich heller als die der silberfreien Palladiumlegierungen. Ihr Nachteil ist es jedoch, daß sie die Verblendkeramik während des Aufbrennprozesses gelb bzw. gelbgrün verfärben. Ursache hierfür ist das Silber, das durch Diffusion bzw. über die Dampfphase in die Keramik gelangt. Es sind bereits mehrere Ansätze unternommen worden, durch entsprechende Legierungskonzeptionen die Verfärbungsneigung von Palladium-Silberlegierungen zu verhindern oder zumindest zu reduzieren.
So wird in der DE-PS 25 23 971 die Zugabe von Titan in der Größenordnung von 0,1-0,5 % und in dem US-Patent 4,350,526 die Zugabe von 0,1 bis 1 % Silizium zu diesem Zweck vorgeschlagen.

Beide Lösungswege sind mit erheblichen Nachteilen bezüglich des zahntechnischen Verarbeitungsverhaltens verbunden. So verursachen beide Elemente eine starke Haftung der Einbettmasse an den Gußopjekten, was das Ausbetten und Ausarbeiten schwieriger und zeitaufwendiger macht.

Palladium bildet mit Silizium darüberhinaus intermetallische Phasen, so daß eine starke Versprödung der Legierung und Gußbrüchigkeit auftreten können. Außerdem besitzen sowohl Titan als auch Silizium eine hohe Reaktivität zum Luftsauerstoff, so daß die Schmelze relativ schnell an diesen Elementen verarmt und damit die beabsichtigte Wirkung reduziert wird. Dies ist besonders bei der Verwendung von Altmaterial (Angußkanäle, Gußtrichter) der Fall.

In der DE-PS 39 05 987 sind Palladium-Silberlegierungen beschrieben, bei denen Unempfindlichkeit gegen Verfärbung erreicht wird, wenn das Element Germanium zusammen mit Gallium und/oder Kobalt in bestimmten Mengenanteilen vorhanden ist. Bei ungünstigen geometrischen Verhältnissen und an den Verblendungsrändern zeigen verfärbungsempfindliche Keramiken jedoch auch dann noch Anzeichen von Verfärbung. Außerdem zeigt es sich, daß am Markt grundsätzliche Vorbehalte gegenüber silberhaltigen Palladiumlegierungen bestehen, auch wenn die Verfärbungsneigung gering oder nicht vorhanden ist.

Die DE-PS 31 46 794 offenbart Edelmetall-Legierungen zum Aufbrennen von Dentalporzellanen, die neben 20 bis 85 Gew. % Palladium, 0 bis 55 Gew. % Gold, 0 bis 40 Gew. % Silber, 1 bis 15 Gew.- % Zinn und/oder Indium und 0,1 bis 3 % Gallium zur Verbesserung der Bruchdehnung und der Gußbrüchigkeit noch je 0,05 bis 1 Gew.-% eines oder mehrerer der Elemente Tantal, Wolfram und Yttrium enthalten.

Tantal und Yttrium haben jedoch den Nachteil, daß sie sehr leicht oxidieren und es daher im Keramiktiegel zu einer Schlackenbildung kommt. Wolfram bildet ein flüchtiges Oxid und stört beim Schmelzen durch Rauchbildung und durch Porenbildung im Gußgefüge.

In der DE-A-3 414 575 wird eine Dentallegierung auf Palladiumbasis beschrieben, die 8 bis 25 Gew.-% Gold, 0 bis 10 Gew.-% Gallium, 0,1 bis 0,5 Gew.-% Rhenium, 0 bis 20 Gew.-% Indium und/oder Zinn, 0 bis 4 Gew.-% Zink und zwingend 0,05 bis 0,25 Gew.-% Bor enthält. Es hat sich jedoch herausgestellt, daß auch sehr geringe Bormengen in solchen Legierungen zur Bildung niedrigschmelzender Phasen an den Korngrenzen führen, was die Temperaturstabilität der gegossenen Teile während des Keramikbrands negativ beeinflußt und zu Paßungenauigkeiten der prothesenteile führt. Außerdem kann Bor auch in kleinsten Mengen einen Verlust der Kornfeinung bedingen und das Gefüge korrosionsanfällig machen.

Es war daher Aufgabe der vorliegenden Erfindung, eine Palladiumlegierung zur Herstellung von mit Dentalkeramik verblendbarem Zahnersatz zu entwickeln, die kupfer-, tantal-, wolfram-, bor- und yttriumfrei ist und nach dem Keramikbrand keine störenden, unästhetischen Verfärbungen zeigt.

Diese Aufgabe wird erfindungsgemäß durch die Verwendung einer Palladiumlegierung mit 66 bis 85 Gew.-% Palladium, 1 bis 20 Gew.% Gold, 0 bis 4 Gew.% Silber, 0,5 bis 4 Gew.% Platin, Eisen und/oder Kobalt, je 0,5 bis 7 Gew.% Gallium, Zinn und Indium, wobei die Summe dieser Gehalte zwischen 9 und 14 Gew.% liegen muß, 0 bis 2 Gew.% Germanium und/oder Zink und 0 bis 1 Gew.% Iridium, Ruthenium und/oder Rhenium gelöst.

Palladiumlegierungen, die kein Silber oder nur geringe Anteile an Silber enthalten, sind generell nur dann zur zahntechnischen Verarbeitung geeignet, wenn sie weitere Nichtedelmetalle enthalten, die das Schmelzintervall absenken und den Wärmeausdehnungskoeffizienten (WAK) anheben. Nur dann lassen sich die Legierungen mit den konventionellen Gießgeräten vergießen und mit herkömmlichen Dentalkeramiken verblenden.

Im allgemeinen führen diese Nichtedelmetalle jedoch dazu, daß die Oxidschicht der Legierungen sehr dunkel wird, so daß die bekannten ästhetischen Probleme auftreten. Überraschenderweise hat sich nun gezeigt, daß deutlich hellere Oxidschichten auftreten, wenn die Nichtedelmetalle Gallium, Zinn und Indium gleichzeitig in Mengen von je 1,5 bis 7 Gew. % und insgesamt in einer Menge von 9 bis 14 Gew. % in der Legierung vorhanden sind. Die Elemente Silber, Gold, Platin, Germanium, Zink, Eisen und Kobalt dienen der Abstimmung der physikalischen Eigenschaften der Legierung, wie z.B. Festigkeit, Härte. Gießbarkeit, thermischer Ausdehnungskoeffizient und Schmelzintervall.

Ruthenium, Rhenium und/oder Iridium werden in Konzentrationen zwischen 0 und 1 Gew. % als Kornfeinungszusätze zulegiert. Versuche mit Verblendkeramiken verschiedener Art haben außerdem gezeigt, daß bei dieser Legierungskonzeption auch ein geringer Silberanteil bis etwa 4 Gew. % keinerlei Verfärbung mehr hervorruft.

Da silberhaltige Palladiumlegierungen von vielen Zahntechnikern mit großem Vorbehalt betrachtet werden, war es wünschenswert, auch absolut silberfreie Palladiumlegierungen mit hellem Oxid herzustellen. Die Herausnahme von Silber führt zunächst zu einer deutlichen Oxidverdunklung und zu einer Anhebung der Schmelztemperatur. Es zeigt sich jedoch, daß durch eine Anhebung des Gallium-Gehaltes auf mindestens 3,1 Gew. % das Schmelzintervall wieder abgesenkt werden kann.

Durch einen Mindestgehalt an Eisen von 1 Gew. % ist wieder ein sehr helles Oxid zu erreichen. Zudem führt der Eisenanteil zu einer wünschenwerten Absenkung des Schmelzintervalls und zu einer Anhebung des WAK, so daß die Absenkung des WAK durch die Herausnahme des Silbers wieder kompensiert wird. Der Eisengehalt darf auf der anderen Seite jedoch nicht zu hoch werden, da es sonst beim Vergießen mit der offenen Flamme zu einer starken Oxidbildung kommt. Diese Oxidationserscheinungen führen außerdem zu blasigen, porösen Gußkegeln. Durch Zugabe geringer Mengen an Germanium und/oder Zink kann diese Erscheinung deutlich reduziert werden.

Eisen- sowie auch Kobaltgehalte in der Legierung führen beim Guß zu einer Braun- bzw. Blaufärbung der Einbettmasse an den Oberflächen, die mit dem flüssigen Metall in Kontakt kommen.

Auf Qualität und Verarbeitung des zahntechnischen Gusses hat dies keinerlei Einfluß. Trotzdem wird diese Verfärbung mit Vorbehalt betrachtet. Der Verzicht auf Eisen und Kobalt kann allerdings zu inakzeptabel hohen Schmelzintervallen und sehr niedrigen WAK-Werten führen. Es zeigte sich jedoch, daß ab einem Mindestgehalt an Gold von 10 Gew. % und Galliumgehalten ab 3,1 Gew. % wieder Palladiumlegierungen mit hellem Oxid und gutem zahntechnischen Verarbeitungsverhalten herstellbar sind.

Besonders bei relativ hohen Goldgehalten neigen eisen- und kobaltfreie Legierungen zu etwas geringeren Härtewerten und grobkörnigerem Gefüge.

Obwohl diese Härtewerte noch im Bereich der Härte von hochgoldhaltigen Aufbrennlegierungen liegen, werden, bedingt durch den verbreiteten Einsatz von Palladiumbasislegierungen, heute im allgemeinen hohe Härtewerte bevorzugt. In dieser Hinsicht hat sich überraschenderweise die Zulegierung von Platin besonders bewährt. Durch die Zugabe von Platin wird die Härte gesteigert, wobei es nicht zu einer Anhebung des Schmelzintervalles kommt, wenn der Platingehalt auf Kosten des Palladiumgehaltes erhöht wird. Zusätzlich wirkt sich Platin günstig auf die Feinheit des Gefüges aus, so daß der Anteil des Kornfeinungsmittels nicht erhöht werden muß. Damit besitzt Platin einen doppelten positiven Einfluß auf die Polierbarkeit der Legierungen. Zum einen wirkt sich die hohe Härte in diesem Fall günstig aus und zum anderen läßt sich der Anteil des Kornfeinungsmittels durch die Wirkung des Platins auf geringem Niveau halten, so daß die Polierbarkeit nicht durch harte Kornfeinungspartikel verschlechtert wird.

Tabelle 1 zeigt die Zusammensetzung einiger beispielhafter erfindungsgemäßen Legierungen, Tabelle 2 deren Eigenschaften und einen Oxidfarbenvergleich mit bekannten kupferhaltigen Palladiumlegierungen bzw. Palladium-Silber-Legierungen.

**Tabelle 1**

| Legierung | Gehalte [Gew. % ] | | | | | | | | | | | |
|---|---|---|---|---|---|---|---|---|---|---|---|---|
| | Pd | Ag | Au | Pt | Fe | Co | Ga | Sn | In | Zn | Ge | Ru |
| 1 | 79,5 | - | 5,5 | | 3 | - | 5 | 4 | 2 | 0,5 | - | 0,50 |
| 2 | 77 | 2 | 5,5 | | - | 3 | 4 | 4 | 4 | - | - | 0,5 |
| 3 | 79 | 1 | 5,5 | | - | 2 | 4 | 4 | 4 | - | - | 0,5 |
| 4 | 76 | 3 | 5,5 | | - | 2 | 4 | 4 | 4 | 1 | - | 0,5 |
| 5 | 74,5 | 2 | 9 | | - | 2 | 4 | 4 | 4 | - | - | 0,5 |
| 6 | 81,5 | - | 2,5 | | 3 | - | 4 | 4 | 4 | - | 0,5 | 0,5 |
| 7 | 71 | - | 15 | 1,4 | - | - | 6 | 4 | 2 | - | 0,1 | 0,5 |
| 8 | 79,5 | 2 | 2,5 | | - | 3 | 4 | 4 | 4 | - | 0,5 | 0,5 |
| 9 | 66,5 | - | 17,5 | 3 | - | - | 6,5 | 4 | 1,5 | 0,5 | | 0,5 |

**Tabelle 2**

| Legierung | Schmelzintervall [°C] | Härte nach Guß [HV5] | Härte nach Brand [HV5] | Oxidfarbenvergleich | |
|---|---|---|---|---|---|
| | | | | Cu-haltige Pd-legierung | Ag-haltige Pd-legierung |
| 1 | 1298-1209 | 252 | 247 | + | + |
| 2 | 1317-1194 | 226 | 220 | + | + |
| 3 | 1331-1231 | 203 | 210 | + | o |
| 4 | 1302-1223 | 217 | 222 | + | + |
| 5 | 1328-1233 | 234 | 230 | + | o |
| 6 | 1321-1218 | 254 | 239 | + | o |
| 7 | 1295-1190 | 261 | 250 | + | o |
| 8 | 1312-1221 | 230 | 221 | + | + |
| 9 | 1286-1182 | 262 | 253 | + | o |
| + = besser o = gleichgut | | | | | |

Die Bestimmung der Oxidfarbe erfolgte im direkten Vergleich mit üblichen silberfreien Palladium-Basislegierungen, die Kupfer enthalten, und mit Palladium-Silber-Legierungen. Die üblichen zahntechnischen Verarbeitungsschritte wurden bei der Probenherstellung möglichst exakt nachgestellt: Nach dem zahntechnischen Guß wurden die Einzelkronen ausgebettet, mit Aluminiumoxid (110 µm) abgestrahlt und anschließend einem simulierten Keramikbrand unterzogen, bei dem die Proben allen Behandlungsschritten, vom Oxidbrand bis zum Glanzbrand, ausgesetzt waren, ohne daß jedoch eine Keramik aufgetragen wurde. Anschließend wurde die so erhaltene Oxidfarbe unter einer Tageslichtleuchte, im direkten Vergleich mit den zwei Standardproben, von zwei Personen bewertet.

Folgende Bewertungsstufen wurden vergeben: Oxidfarbe gleich, bzw. heller oder dunkler (in der Tabelle 2 mit "0", "+", "-" bezeichnet), als die jeweilige Vergleichsprobe. Alle erfindungsgemäßen Legierungen zeigen ein helleres Oxid als die üblichen silberfreien, aber kupferhaltigen Palladiumlegierungen, so daß in dieser Spalte nur "+" steht. Im Vergleich zur Palladium-Silber-Legierung sind sowohl hellere als auch gleiche Oxidfarben feststellbar.

## Patentansprüche

1. Verwendung einer kupfer-, tantal-, wolfram- und yttriumfreien Palladiumlegierung aus 66 bis 85 Gew.% Palladium, 1 bis 20 Gew.% Gold, 0 bis 4 Gew.% Silber, 0,5 bis 4 Gew.% Platin, Eisen und/oder Kobalt, je 0,5 bis 7 Gew.% Gallium, Zinn und Indium, wobei die Summe dieser Gehalte zwischen 9 und 14 Gew.% liegen muß, 0 bis 2 Gew.% Germanium und/oder Zink und 0 bis 1 Gew.% Iridium, Ruthenium und/oder Rhenium zur Herstellung von mit Dentalkeramik verblendbarem Zahnersatz.

## Claims

1. Use of a palladium alloy containing no copper, tantalum, tungsten and yttrium prepared from 66 to 85 wt.% of palladium, 1 to 20 wt.% of gold, 0 to 4 wt.% of silver, 0.5 to 4 wt.% of platinum, iron and/or cobalt, 0.5 to 7 wt.% of each of gallium, tin and indium, wherein the sum of these contents must be between 9 and 14 wt.%, 0 to 2 wt.% of germanium and/or zinc and 0 to 1 wt.% of iridium, ruthenium and/or rhenium for the production of a dental prosthesis which may be overlaid with dental ceramic.

## Revendications

1. Utilisation d'un alliage de palladium exempt de cuivre, de tantale, de tungstène et d'yttrium à base de 66 à 85 % en poids de palladium, 1 à 20 % en poids d'or, 0 à 4 % en poids d'argent 0,5 à 4 % en poids de platine, de fer et/ou de cobalt, avec 0,5 à 7 % en poids de gallium, d'étain et d'indium, où la somme de ces teneurs doit être comprise entre 9 et 14 % en poids, 0 à 2 % en poids de germanium et/ou de zinc et 0 à 1 % en poids d'iridium, de ruthénium et/ou de rhénium pour produire des prothèses dentaires qu'on peut doubler de céramique dentaire.
